# EUROPEAN PATENT SPECIFICATION

(11) **EP 4 299 109 B1**
(45) Date of publication and mention of the grant of the patent: **13.08.2025**
(21) Application number: 23178820.9
(22) Date of filing: 13.06.2023
(51) Int. Cl.: A61N 1/372, G16H 40/67

(54) **SYSTEM FOR IMPLANTABLE MEDICAL DEVICE REMOTE PROGRAMMING**
SYSTEM ZUR FERNPROGRAMMIERUNG EINER IMPLANTIERBAREN MEDIZINISCHEN VORRICHTUNG
SYSTÈME DE PROGRAMMATION À DISTANCE DE DISPOSITIF MÉDICAL IMPLANTABLE

(30) Priority: 27.06.2022 US 202263367064 P; 21.10.2022 US 202263380420 P
(43) Date of publication of application: 03.01.2024
(73) Proprietor: Pacesetter, Inc., Sylmar, CA 91342 (US)
(72) Inventor: JIN, Maobing, Sylmar, 91342 (US); NARANG, Nidhi, Sylmar, 91342 (US); SELVERIAN, Voltaire, Sylmar, 91342 (US); BISEN, Pulkit, Sylmar, 91342 (US); KANG, Chinsoo, Sylmar, 91342 (US)
(74) Representative: Barker Brettell Sweden AB

(56) References cited:
- US-A1- 2020 372 996
- US-A1- 2021 052 900
- US-B2- 10 967 190
- US-B2- 9 878 166

## Description

### TECHNICAL FIELD

Embodiments of the present disclosure generally relate to methods, assemblies, systems, and devices for updating operating configurations of implantable medical devices (IMDs) via remote communications.

### BACKGROUND

An IMD is a medical device that is configured to be implanted within a patient anatomy and commonly employs one or more electrodes that either receive or deliver voltage, current or other electromagnetic pulses from or to an organ or tissue for diagnostic or therapeutic purposes. In general, IMDs include a battery, electronic circuitry, a pulse generator, a transceiver, and a microprocessor. The microprocessor is configured to handle communication with an external device or instrument as well as control patient therapy. The components of the IMD are hermetically sealed within a housing. The IMD is completely enclosed within the human body. Thus, there is no means of direct interaction between an IMD and an external device, other than through wireless communication.

The IMD may require programming updates over time to adjust the behavior and operations of the IMD. The programming updates are wirelessly communicated from an external device outside of the patient to the IMD within the patient. When updating the IMD, care must be taken to ensure that the software, firmware, application, program, or the like that is being updated is the software, firmware, application, program, or the like of the update. For example, when a patient experiences a health issue with their IMD and goes to a hospital, a clinician at the hospital can change a treatment, updates the software, firmware, application, program, or the like of the IMD. If the patient then goes to their regular clinician, and the regular clinician has a different treatment, or update they desire to implement, it is important to the regular clinician to know the current treatment, updates to the software, firmware, application, program, etc. before making such a change. Otherwise, mistakes can occur related to the update, potentially harming the patient.

A need remains for improved administration of remote programming communications to update an IMD.

US 9,878,166 discloses a method, computer medium, and system for programming a controller. The controller controls electrical stimulation energy output to electrodes, and stores a set of programmed stimulation parameters associated with the electrodes. The programmed stimulation parameter set is compared with sets of reference stimulation parameters, each of the reference sets of stimulation parameters being associated with the electrodes. If an identical match is determined between the programmed stimulation parameter set and any one of the reference stimulation parameter sets exists based on the comparison, the identically matched stimulation parameter set is selected as an initial stimulation parameter set. If an identical match does not exist, a best between the programmed stimulation parameter set and the reference stimulation parameter sets is determined and selected as the initial stimulation parameter set. The controller is then programmed with a new set of programmable stimulation parameters based on the initial stimulation parameter set.

US 10,967,190 discloses a method for operating a system for management of implantable medical devices (IMDs). The method comprises conducting communications sessions with a plurality of clinician programmer devices, wherein some of the communication sessions occur while the plurality of clinician programmer devices are engaged in respective programming sessions with IMDs, conducting communications sessions with a plurality of patient controller devices, wherein the communication sessions with the patient controller devices include communication of data pertaining to offline programming of IMDs, reconciling programming session data received from the plurality of clinician programmer devices with programming session data received from patient controller devices to identify instances of unauthorized IMD programming, and distributing revocation data to patient controller devices to be downloaded to corresponding IMDs, wherein the revocation data identifies cryptographic keys that are no longer trusted.

### SUMMARY

The present invention is defined in the independent claims. Further embodiments of the invention are defined in the dependent claims.

In accordance with an embodiment, a medical device assembly is provided that includes a remote programming engine and a clinician programing device configured to communicate with an implanted medical device of a patient. The clinician programming device includes one or more processors configured to communicate program instructions to the remote programming engine. The remote programming engine includes one or more processors configured to simulate operation of the implanted medical device based on the updated program instructions, and communicate updated program instructions to the implanted medical device based on the operation simulated.

Optionally, to simulate operation of the implanted medical device, the one or more processors of the remote programming engine are further configured to determine initialization of the programming session, whether the programming session is pending, and termination of the programming session. In one aspect, the one or more processors of the remote programming engine are further configured to determine whether the termination of the programming session is in response to completion of the programming session, or in response to a programming error. Optionally, to determine initialization of the programming session, the one or more processors of the remote programming engine are further configured to generate an asynchronous remote programming profile that includes the updated program instructions, and communicate the asynchronous remote programing profile to the implanted medical device. In yet another aspect, the one or more processors of the clinician processing device are further configured to generate and display a pending message in response to communication of the asynchronous remote programing profile, and present the pending message until an acknowledgment message is received from a patient application. Optionally, the one or more processors of the clinician processing device are further configured to display a success message in response to receiving the acknowledgment message. Alternatively, the one or more processors of the remote programming engine are further configured to start a timer in response to communicating the asynchronous remote programing profile, and communicate a failure message in response to the timer reaching a determined period before receiving the acknowledgment message.

In accordance with an embodiment, a method for updating instructions for an implanted medical device under control of one or more processors is provided. The method includes communicating updated program instructions related to a programming session of the implanted medical device, simulating operation of the implanted medical device based on the updated program instructions, and communicating the updated program instructions to the implanted medical device based on the operation simulated.

Optionally, simulating operation of the implanted medical device includes initializing an updated programming session, determining whether the updated programming session is pending, and terminating of the updated programming session. In one aspect, initializing the updated programming session includes communicating an update message to a virtual device engine (VDE), the message including the updated program instructions for the updated programming session that is related to a treatment to be performed by the implanted medical device, accessing a patient profile in response to receiving the update message at the virtual device engine, generating an asynchronous remote programming profile that includes the updated program instructions, and communicating the asynchronous remote programing profile to the implanted medical device. In yet another aspect, the method also includes generating and displaying a pending message in response to communicating the asynchronous remote programing profile, and presenting the pending message until an acknowledgment message is received from a patient application. In one example, the method also includes displaying a success message in response to receiving the acknowledgment message. In another example, the method also includes starting a timer in response to communicating the asynchronous remote programing profile, and displaying a failure message in response to the timer reaching a determined period before receiving the acknowledgment message. In yet another example, the method additionally includes logging operation of programming sessions within a virtual device engine database in response to the timer reaching the determine period before receiving the acknowledgment message.

In an example embodiment a computer program product comprising a non-signal computer readable storage medium is provided, including computer executable code to obtain updated program instructions related to a programming session of an implanted medical device, simulate operation of the implanted medical device based on the updated program instructions, and communicate the updated program instructions to the implanted medical device based on the operation simulated.

Optionally, to simulate operation of the implanted medical device, the computer executable code to determine initialization of the programming session, whether the programming session is pending, and termination of the programming session. In one aspect, computer executable code to additionally determine whether the termination of the programming session is in response to completion of the programming session, or in response to a programming error. In another aspect, to determine initialization of the programming session, the computer executable code also to access a patient profile at a virtual device engine (VDE) in response to obtaining the updated program instructions, generate an asynchronous remote programming profile that includes the updated program instructions, and communicate the asynchronous remote programing profile to the implanted medical device. In one example, the computer executable code additionally to generate a pending message in response to communicating the asynchronous remote programing profile, and present the pending message until an acknowledgment message is received from a patient application. In another example, the computer executable code also to communicate a success message to a clinician application in response to receiving the acknowledgment message.

In one example embodiment, a a clinician programing device configured to communicate with an implanted medical device of a patient is provided. The clinician programming device includes one or more processors configured to communicate program instructions related to a programming session of the implanted medical device to a remote programming engine, receive updated program instructions from the remote programming engine based on a simulation of operation of the implanted medical device based on the program instructions, and communicate updated program instructions to the implanted medical device based on the updated program instructions.

Optionally, the one or more processors are configured to receive communication related to initialization of the implantable medical device, related to whether the programming session is pending, and related to termination of the programming session. In one aspect, the one or more processors are additionally configured to determine whether the termination of the programming session is in response to completion of the programming session, or in response to a programming error. In another aspect the one or more processors are further configured to receive a message from a virtual device engine (VDE), the message including the updated program instructions for the programming session that is related to a treatment to be performed by the implanted medical device, receive an asynchronous remote programming profile from the VDE that includes the updated program instructions, and communicate the asynchronous remote programing profile to the implanted medical device. In one example, the one or more processors further configured to generate and display a pending message in response to communicating the asynchronous remote programing profile, and present the pending message until an acknowledgment message is received from a patient application. In another example, the one or more processors further configured to display a success message in response to receiving the acknowledgment message. In yet another example, the one or more processors further configured to display a failure message in response to a timer reaching a determined period before receiving the acknowledgment message.

### BRIEF DESCRIPTION OF THE DRAWINGS

Figure 1 illustrates a simplified block diagram of a system operated in accordance with embodiments herein.
Figure 2 shows a block diagram of the system of Figure 1, according to an embodiment.
Figure 3 is a schematic illustration of a system, according to an embodiment.
Figure 4 is a schematic block diagram of a system for verifying an update sent by a clinician, according to an embodiment.
Figure 5 is a schematic block flow diagram of a process for managing a programming session, according to an embodiment.
Figure 6 is a schematic block flow diagram of process for initializing an IMD, in accordance to an embodiment.
Figure 7 is a schematic block flow diagram for updating and IMD, in accordance to an embodiment.
Figure 8 is a schematic block flow diagram for a process for providing programming updates for an IMD, in accordance to an embodiment.
Figure 9 is a schematic block flow diagram for a process updating an IMD, in accordance to an embodiment.
Figure 10 is a schematic block flow diagram for using a timer for updating an IMD, in accordance to an embodiment.

### DETAILED DESCRIPTION

Embodiments herein provide methods, assemblies, systems and devices that are designed to enhance integrity and authenticity of wireless programming communications between one or more external devices and an IMD.

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The term "clinician programming device", as used herein refers to any and all electronic devices utilized by a clinician, technician, physician, caregiver, nurse, doctor, or the like. The electronic device can be a central processing unit (CPU), desktop computer, laptop computer, tablet, smartphone, smart watch, monitor console, etc. The electronic device includes one or more processors configured to follow instructions, and a transceiver configured to communicate over a network, in a cloud, wirelessly, over the air, through a wire, or the like. In one example, the clinician programing device communicates with a patient programming device.

The term "patient programming device", as used herein refers to any and all electronic devices utilized by a patient to monitor or make determination related to the IMD of the patient. The electronic device can be a central processing unit (CPU), desktop computer, laptop computer, tablet, smartphone, smart watch, monitor console, etc. The electronic device includes one or more processors configured to follow instructions, and a transceiver configured to communicate over a network, in a cloud, wirelessly, over the air, through a wire, or the like. In one example, the patient programing device communicates with a clinician programming device.

The term "programming session" is any period of time dedicated to operation of an IMD based a set of instruction to provide a treatment. The set of instructions can be stored in a storage device of the IMD, or communicated to the IMD. The set of instructions can include a treatment, pacing pattern, etc. for the IMD, and the IMD operates during the programming session based on those instructions. The programming session begins with the program instructions that are provided, uploaded, communicated, etc. (e.g., updated program instructions) and the IMD begins operating based on the set of instructions. The programming session ends when the IMD stops using the set of instructions for operation. In example embodiments, the IMD can terminate a session as a result of receiving a new set of instructions, an updated set of instructions, a change to the set of instructions, varying the set of instructions, or the like.

The term "simulate operation" as used here in relation to an IMD refers to producing a computer model of the operation of the IMD based on the current set of instructions that the IMD has, combined with any changes, updates, etc. of the instructions requested. By creating the computer model, a check can be provided before communicating the update, changes, etc. to ensure the update, changes, etc. are to the most recent set of instructions at and IMD and will not cause error.

The term "initialization" as used herein refers to the preparation of an electronic device for operation in which diagnostic test are run, and the operating system is loaded. The initialization of an electronic device must occur before any changes, updates, or the like can be installed, provided, communicated, etc. to the operating system of the electronic device.

The term "virtual device engine" (VDE) as used herein refers to a simulation, or computer model stored within a cloud, network, or the like that can be utilized to represent an IMD. The VDE can be utilized to replicate, or simulate the operation of the IMD, including how an update, change, etc. to a programming session, or set of instructions will occur at an IMD prior to the update, change, etc. being communicated to the IMD. In this manner the VDE can be utilized to adjust, change, update, etc. the update, change, etc. to be communicated based on the VDE.

The term "asynchronous remote programming profile" as used herein refers to unique, custom, or varied program instructions (e.g., updated program instructions) for an IMD of an individual patient formed at a location other than within the IMD. The asynchronous remote programming profile includes any changes, variations, updates, or the like to update program instructions that are made as a result of simulations made from the VDE.

The term "obtains" and "obtaining", as used herein in connection with data, signals, information, and the like, include at least one of i) accessing memory of an external device or remote server where the data, signals, information, etc. are stored, ii) receiving the data, signals, information, etc. over a wireless communications link between the IMD and a local external device, and/or iii) receiving the data, signals, information, etc. at a remote server over a network connection. The obtaining operation, when from the perspective of an IMD, may include sensing new signals in real time, and/or accessing memory to read stored data, signals, information, etc. from memory within the IMD. The obtaining operation, when from the perspective of a local external device, includes receiving the data, signals, information, etc. at a transceiver of the local external device where the data, signals, information, etc. are transmitted from an IMD and/or a remote server. The obtaining operation may be from the perspective of a remote server, such as when receiving the data, signals, information, etc. at a network interface from a local external device and/or directly from an IMD. The remote server may also obtain the data, signals, information, etc. from local memory and/or from other memory, such as within a cloud storage environment and/or from the memory of a workstation or clinician external programmer.

Provided is medical device assembly for remotely programming an IMD of a patient. The medical device assembly includes a clinician programming device with a clinician application that communicates with an IMD of a patient, and a patient application at a patient programming device over a network. Within the network, and in one example, within a cloud, a virtual device engine (VDE) is provided that receives communications from the IMD related to the programming status of the IMD, including pending updates, updates previously provided, and the like. When a clinician provides updated program instructions for the IMD through the clinician application, the VDE simulates the IMD programming to determine whether updated program instructions are compatible with the current programming of the IMD. Based on the simulation, an asynchronous remote programming profile is generated for updating the IMD. In this manner, the IMD cannot be incorrectly updated by a clinician that does not have knowledge of the current programming status of the IMD.

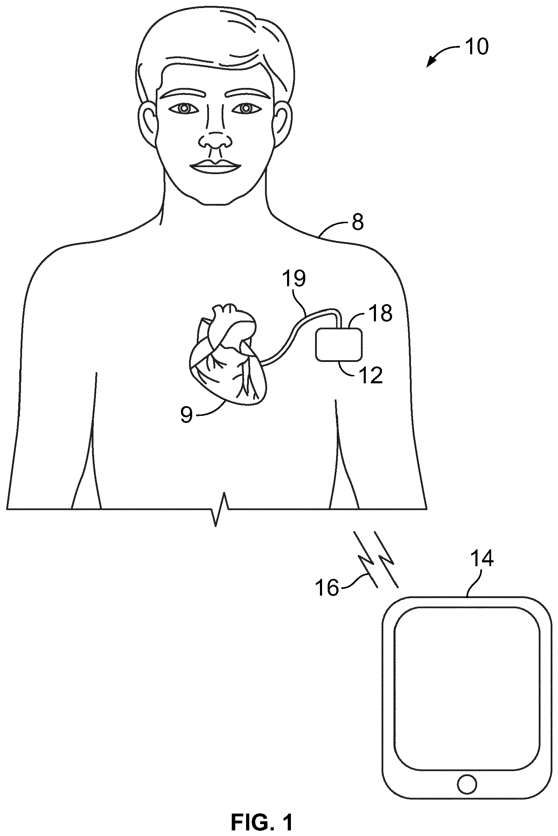

Figure 1 illustrates a simplified block diagram of a system 10, also referred to as assembly herein, operated in accordance with embodiments herein. The system 10 includes an IMD 12 and one or more external devices or instruments. A single external device 14 is illustrated in Figure 1. The IMD 12 and the external device 14 are configured to communicate with one another wirelessly over a wireless communication link 16. In an embodiment, the external device 14 updates an operating configuration of the IMD 12 by transmitting a programming package to the IMD 12 along the communication link 16. The embodiments described herein provide an algorithm or workflow for verifying the validity, integrity, and authenticity of configuration change requests of the programming package, including a set of instructions of the programming package, prior to executing the configuration change requests to update the operating configuration (e.g. programming session) of the IMD. For example, a configuration change for an implantable pacemaker or cardioverter defibrillator may represent a change in sensitivity threshold, a change in heart rate thresholds for binning atrial fibrillation or tachycardia events, a change in the number of arrhythmia events that should occur before delivering a therapy, a change in communications gain, a change in a communications protocol, or the like. A configuration change of a programming session may include updates to executing operations of importance for diagnosis and/or clinical care, such as lead impedance testing, testing for level of sensing, or even disabling device operations as part of palliative care.

The IMD 12 is implanted within a patient 8 at a site near the heart 9. The IMD 12 may be a cardiac pacemaker, an implantable cardiac monitoring device (ICM), a defibrillator, an ICM coupled with a pacemaker, or the like. The IMD 12 is intended for implantation within a subcutaneous pocket of the patient. The IMD 12 may be configured to sense cardiac signals to monitor cardiac activity over time. Optionally, the IMD 12 may also be configured to deliver stimulation therapy to the heart 9. For example, the IMD 12 may be a dual-chamber stimulation device capable of treating both fast and slow arrhythmias with stimulation therapy, including cardioversion, defibrillation, and pacing stimulation, as well as capable of detecting heart failure, evaluating its severity, tracking the progression thereof, and controlling the delivery of therapy and warnings in response thereto.

The IMD 12 in the illustrated embodiment includes a body or housing 18 that is connected to at least one lead 19. A single lead 19 is shown in Figure 1, but the IMD 12 may include multiple leads in another embodiment. The lead 19 extends from the housing 18 to the heart 9, such that the distal end is in contact with patient tissue surrounding the heart 9. The lead 19 includes one or more electrodes that may measure cardiac signals of the heart 9 and deliver stimulation therapy to the heart 9. In an embodiment, a single electrode may emit a stimulation pulse in a stimulation mode, and then may quickly switch to a monitoring mode to detect cardiac signals following the stimulation pulse.

Although the IMD 12 in the illustrated embodiment includes a lead 19, one or more of the embodiments described herein utilize a leadless IMD that does not include any lead. For example, the IMD 12 may be a leadless pacemaker, a leadless cardiac monitoring device (ICM), or the like. In general, the IMD 12 may represent a cardiac monitoring device, pacemaker, cardioverter, cardiac rhythm management device, defibrillator, neurostimulator, leadless monitoring device, leadless pacemaker, and the like. For example, the IMD may include one or more structural and/or functional aspects of the device(s) described in U.S. Patent 9,333,351 "Neurostimulation Method And System To Treat Apnea"; U.S. Patent 9,044,610 "System And Methods For Providing A Distributed Virtual Stimulation Cathode For Use With An Implantable Neurostimulation System"; U.S. Patent 10,765,860 "Subcutaneous Implantation Medical Device With Multiple Parasternal-Anterior Electrodes"; U.S. Patent 10,722,704 "Implantable Medical Systems And Methods Including Pulse Generators And Leads"; and U.S. Patent 11,045,643 "Single Site Implantation Methods For Medical Devices Having Multiple Leads". Additionally or alternatively, the IMD may be a leadless implantable medical device (LIMD) that include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device". Additionally or alternatively, embodiments may be implemented utilizing all or portions of the methods and systems described in U.S. Patent Publication Number 2021/0020294 "Methods, Devices And Systems For Holistic Integrated Healthcare Patient Management".

In another example, the IMD 12 may be an ICM that includes one or more structural and/or functional aspects of the device(s) described in U.S. Patent 9,949,660 "Method And System To Discriminate Rhythm Patterns In Cardiac Activity".

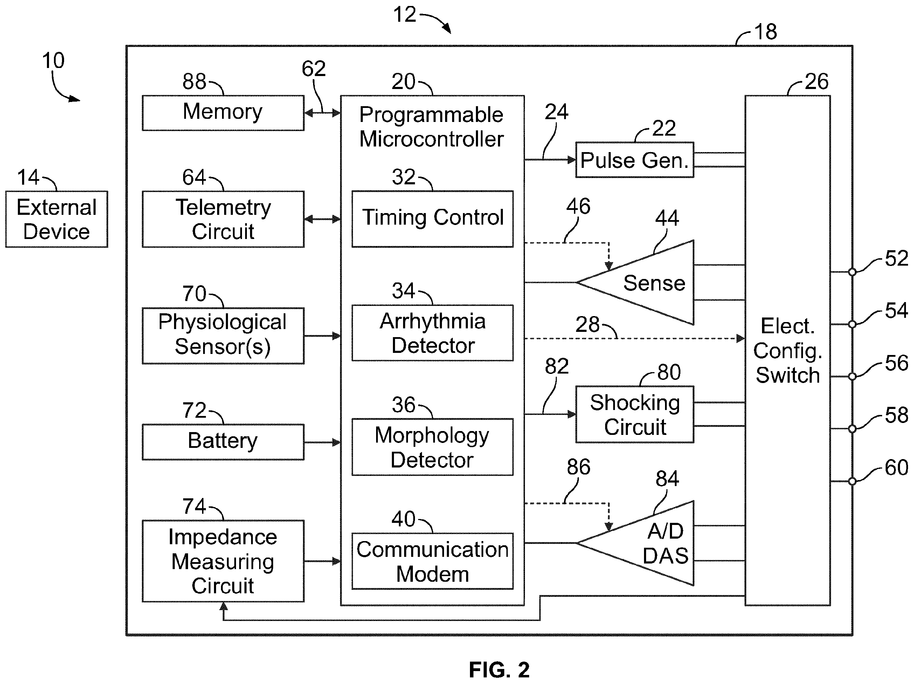

The housing 18 may contain a battery, pulse generation circuitry, communication circuitry, a data storage device (e.g., memory), and/or control circuitry including one or more processors. The control circuitry is for receiving and analyzing electrocardiogram IEGM signals from the electrodes 13. The control circuitry may include at least one processor for processing the IEGM signals in accordance with algorithms to make determinations about the state of the heart 9. The memory provides storage for the cardiac signals and programmed instructions for the control circuitry that provide a programming session. The battery powers the circuitry with the housing 10. For example, the battery powers the pulse generation circuitry to generate stimulation pulses and powers the communication circuitry to communicate with an external device 16. The control circuitry may generate messages to be communicated via the communication circuitry to the external device 14. The messages may include the IEGM signals and/or data generated based on the IEGM signals. The control circuitry is also configured to analyze messages, received via the communication circuit from the external device 14, that include programming packages for updating the operating configuration, including settings, parameters, behavior, and the like, of the IMD 12. Exemplary structure for the IMD 12 is discussed and illustrated below in connection with Figure 2.

The external device 14 may represent a computing device that is accessible or possessable by the patient or a clinician, such as a tablet computer, a smartphone, a wearable device, laptop computer, a desktop computer, a bedside monitor installed in a patient's home, or the like. The external device 14 alternatively may be a programmer device used in the clinic by a clinician. The external device may be one of the devices listed above that is communicatively connected to a second external device that represents the source of the programming package intended to update the operating configuration of the IMD 12. For example, the second external device may be a server or another computing device that is communicatively connected to the first external device 14 via a network connection (e.g., the Internet). In general, the external device 14 facilitates access by physicians to patient data as well as permits the physician to review real-time electrocardiogram (ECG) signals and, as specifically described herein, update the operating configuration of the IMD 12 without the clinician being near the patient.

Figure 2 shows a block diagram of the system 10 according to an embodiment. The housing 18 or case of the IMD 12 holds the electronic and/or computing components. The housing 18 further includes a connector (not shown) with at least one terminal 52 and optionally additional terminals 54, 56, 58, 60. The terminals may be connected to electrodes that are located in various locations within and about the heart, such as on the lead 19 (shown in Figure 1). The electrodes may include various combinations of ring, tip, coil, shocking electrodes, and the like.

The IMD 12 includes a programmable microcontroller 20 that controls various operations of the IMD 12, including cardiac monitoring and stimulation therapy. Microcontroller 20 includes a one or more processors (e.g., a microprocessors or equivalent control circuitry), RAM and/or ROM memory, logic and timing circuitry, state machine circuitry, and I/O circuitry. Microcontroller 20 includes an arrhythmia detector 34 that is configured to detect cardiac activity data to identify potential atrial fibrillation (AF) episodes as well as other arrhythmias (e.g., Tachycardias, Bradycardias, Asystole, etc.).

An electrode configuration switch 26 is optionally provided to allow selection of different electrode configurations under the control of the microcontroller 20. The electrode configuration switch 26 may include multiple switches for connecting the desired electrodes to the appropriate I/O circuits, thereby facilitating electrode programmability. The switch 26 is controlled by a control signal 28 from the microcontroller 20. Optionally, the switch 26 may be omitted and the I/O circuits directly connected to a housing electrode.

The IMD 12 may include a chamber pulse generator 22 that generates stimulation pulses for connecting the desired electrodes to the appropriate I/O circuits, thereby facilitating electrode programmability. The pulse generator 22 is controlled by the microcontroller 20 via control signals 24. The IMD 12 includes a sensing circuit 44 selectively coupled to one or more electrodes that perform sensing operations through the switch 26 to detect cardiac activity. The sensing circuit 44 may include dedicated sense amplifiers, multiplexed amplifiers, or shared amplifiers. The sensing circuit 44 may operate in a unipolar sensing configuration or a bipolar sensing configuration. The output of the sensing circuit 44 is connected to the microcontroller 20 which, in turn, triggers, or inhibits the pulse generator 22 in response to the absence or presence of cardiac activity. The sensing circuit 44 receives a control signal 46 from the microcontroller 20 for purposes of controlling the gain, threshold, polarization, and timing of any blocking circuitry (not shown) coupled to the sensing circuit.

The IMD 12 further includes an analog-to-digital A/D data acquisition system (DAS) 84 coupled to one or more electrodes via the switch 26 to sample cardiac signals across any pair of desired electrodes. The A/D DAS 84 is controlled by a control signal 86 from the microcontroller 20.

The IMD 12 is further equipped with a communication modem (modulator/demodulator) or circuit 40 to enable wireless communication. The modem 240 enables timely and accurate data transfer directly from the patient to the external device 14, and vice-versa. For example, the communication modem 40 is configured to establish the communication link 16 with the external device 14. In an embodiment, the communication modem 40 receives a programming package from the external device 14 and conveys the programming package for updating, changing, varying, etc. the programming session to the microcontroller 20 for verification prior to executing configuration change requests and/or command execution requests contained within the programming package. In addition to remote programming of the IMD 12, the wireless communication link 16 with the external device 14 facilitates access by physicians and/or patients to patient data generated by the IMD 12.

The communication modem 40 may utilize radio frequency (RF), Bluetooth, or Bluetooth Low Energy telemetry protocols. The signals are transmitted in a high frequency range and will travel through the body tissue in fluids without stimulating the heart or being felt by the patient. The communication modem 40 may be implemented in hardware as part of the microcontroller 20, or as software/firmware instructions programmed into and executed by the microcontroller 20. Alternatively, the modem 40 may reside separately from the microcontroller 20 as a standalone hardware component.

The microcontroller 20 is coupled to a non-transitory data storage device, referred to herein as memory device 88, by a suitable data/address bus 62. The memory device 88 stores programmable operating parameters used by the microcontroller 20 and/or data associated with the detection and determination of arrhythmias. In an embodiment, the memory device 88 also stores the current programming session, along with any and all changes, modifications, updates, or the like previously made to the programming session.

The IMD 12 optionally includes one or more physiologic sensors 70 that adjust pacing stimulation rates, detect changes in cardiac output, changes in the physiological condition of the heart, and/or diurnal changes in activity (e.g., detecting sleep and wake states). Examples of physiological sensors 70 might include sensors that, for example, sense respiration rate, pH of blood, ventricular gradient, activity, body movement, position/posture, minute ventilation (MV), and/or the like.

The battery 72 provides operating power to all of the components in the IMD 12. The battery 72 is capable of operating at low current drains for long periods of time, and is capable of providing a high-current pulses (for capacitor charging) when the patient requires a shock pulse (e.g., in excess of 2 A, at voltages above 2 V, for periods of 10 seconds or more).

The IMD 12 further includes an impedance measuring circuit 74, which can be used for many things, including sensing respiration phase. The IMD 12 may be further equipped with a telemetry circuit 64 that can selectively communicate with an external device, such as the device 14, when connected via a physical (e.g., wired) communication link. The IMD 12 includes a shocking circuit 80 controlled by control signals 86 generated by the microcontroller 20. The shocking circuit 80 generates shocking pulses of low (e.g., up to 0.5 joules), moderate (e.g., 0.5-10 joules), or high energy (e.g., 11 to 40 joules), as controlled by the microcontroller 20. In an alternative embodiment in which the IMD 12 senses and monitors cardiac activity without administering stimulation therapy, the IMD 12 may lack the pulse generator 22 and the shocking circuit 80.

The microcontroller 20 may include other dedicated circuitry and/or firmware/software components, such as a timing control (module) 32 and a morphology detector (module) 36. The timing control 32 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The morphology detector 36 is configured to review and analyze one or more features of the morphology of cardiac activity signals, such as the morphology of detected R waves to determine whether to include or exclude one or more beats from further analysis.

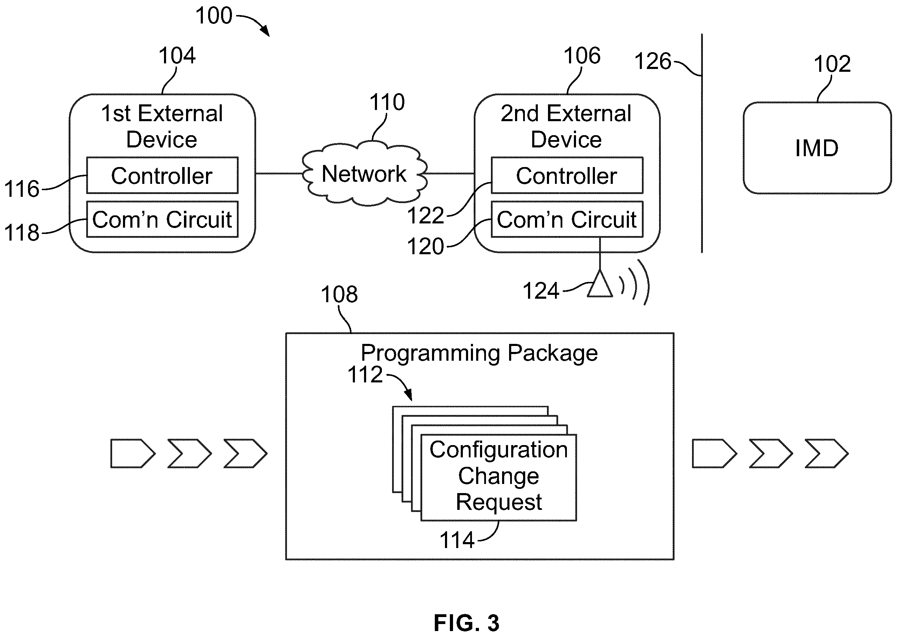

Figure 3 is a schematic illustration of a system 100 according to an embodiment. The system 100 provides secure remote programming of an IMD 102 while the IMD 102 is implanted within a patient. In addition to the IMD 102, the system 100 includes one or more external devices. In the illustrated embodiment, the external devices include a first external device 104 and a second external device 106, but the system 100 may include only a single external device or more than two external devices in other embodiments. The system 100 may represent the system 10 shown in Figures 1 and 2. For example, the IMD 102 may represent the IMD 12 and the second external device 106 may represent the external device 14.

The one or more external devices build a programming package 108 that is designed to update an operating configuration of a programming session of the IMD 102. The operating configuration of the programming session of the IMD 102 generally refers to the specific settings, functions, parameters, and the like that dictate the device behavior. For example, the operating configuration affects how the IMD 102 collects data, such as cardiac signals, how the IMD 102 analyzes the data, and how the IMD 102 responds to the data. The response may include activating the pulse generator or shocking circuit to deliver stimulation therapy to the patient, saving data in the memory device, communicating data to the external device 106, or the like. Even if the IMD 102 has been previously programmed, the operating configuration of the programming session may be updated over time due to physiological changes in the patient, unplanned movement of the IMD 102 within the patient, or even to enhance functionality of the IMD 102 based on software developments.

In the illustrated embodiment, the first external device 104 builds the programming package 108 related to the programming session. In a non-limiting example, the first external device 104 is accessed and utilized by a clinician to select a collection 112 of multiple configuration change requests 114 for inclusion in the programming package 108. The first external device 104 may be a clinician programming device, a workstation, or a computing device (e.g., smartphone, tablet computer, laptop computer, etc.). The clinician may select the collection 112 by communicating via a network 110 with a server or other computing device in the cloud. For example, the clinician may be required log in to a secure portal prior to receiving access to select the configuration change requests 114 and command the generation of the programming package 108. The first external device 104 may be remote from the second external device 106 and the patient.

The first external device 104 conveys the programming package 108, as a message, to the second external device 106 via the network 110. In a non-limiting example, the second external device 106 is a device that is disposed proximate to the patient, at least at a time that the second external device 106 communicates the programming package 108 to the IMD 102. For example, the second external device 104 may be a bedside monitoring device or a computing device utilized or possessed by the patient, such as a smartphone, tablet computer, laptop computer, or the like.

When the second external device 106 receives the programming package 108, the programming package 108 may schedule a designated time in the future at which to transmit the programming package 108 to the IMD 102. The second external device 106 initially stores the programming package 108 in a memory device of the device 106 until the designated time. At the designated time, the second external device 106 wirelessly transmits the programming package 108 to the IMD 102 to update a programming session. This process of scheduling in advance the delivery of the programming package to the IMD via an intermediary device is referred to as asynchronous remote programming. The IMD 102 is implanted within the patient at the time that the IMD 102 receives the programming package 108. According to embodiments herein, the IMD 102 utilizes a virtual device engine (VDE) to simulate operation of the IMD based on the programming package. The VDE communicates with the IMD to ensure the most recent programming configuration is known. In this manner, the VDE can verify whether the programming package is configured and based on the most recent and most updated programming session at the IMD. If all of the elements of the programming package 108 are verified, then the IMD 102 executes all of the configuration change requests 114 to update the operating configuration of the IMD 102. On the other hand, if at least one of the elements are not verified, as described herein, then the IMD 102 does not execute any of the configuration change requests 114, such that the operating configuration of the IMD 102 is not updated based on the change request.

The first and second external devices 104, 106 include respective controller circuits (e.g., controllers) and communication circuits. Each of the controllers includes one or more processors. In the process described above, the controller 116 of the first external device 104 generates the programming package 108. The communication circuit 118 of the first external device 104 communicates the programming package 108 to the second external device 106 via the network 110. The communication circuit 120 of the second external device 106 receives the programming package 108 and conveys the package 108 to the controller 122 of the second external device 106 for analysis. The controller 122 determines the scheduled delivery time and transmission characteristics, such as frequency channel and protocol, for communicating the programming package 108 to the IMD 102. At the scheduled delivery time, the controller 122 utilizes the communication circuit 120 to transmit the programming package 108 to the IMD 102. The communication circuit 120 may include an antenna 124 that wirelessly transmits the programming package 108 via RF signals, such as Bluetooth. The signals that represent the programming package 108 penetrate the body 126 of the patient and reach a receiver of the IMD 102. The receiver may be the communication modem 40 shown in Figure 2, which conveys the received data packets to the one or more processors of the microcontroller 20 for analysis. In this way, the IMD 102 is beneficially able to receive the programming package 108 without requiring a network connection or a proximity sensor.

In instances as described above in which the communication of the programming package 108 from the source to the IMD 102 is delayed and/or indirect, there is a risk that the contents of the programming package 108 may be tampered with or corrupted after generation and before receipt by the IMD 102. The system 100 and method described herein perform cryptographic operations to ensure that the configuration change requests 114 are immutable and that the source of the package 108 is authenticated prior to execution by the IMD 102.

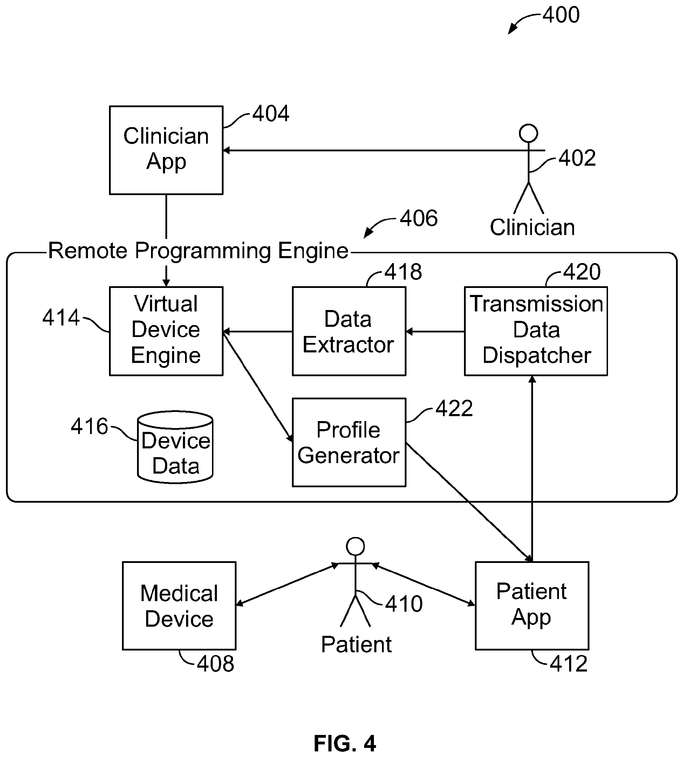

Figure 4 illustrates a system 400 for verifying an update sent by a clinician 402 from a clinician application 404 through a remote programming engine (RPE) 406 to an IMD 408 of a patient 410 that utilizes a patient application 412. The patient application 412 in one example can be accessed and programmed at a patient programming device. The update may be an update of a treatment, software, firmware, application, program, etc. The clinician application 404 may be stored in a storage device of a clinician programming device, including a clinician laptop computer, desktop computer, CPU, smartphone, smartwatch, tablet, or the like. The clinician application 404 may also be stored within a network, such as in the cloud, at a server, at a network resource, or the like. In each instance, the clinician application includes instructions that may be executed by one or more processors. The one or more processors can be of the clinician laptop computer, desktop computer, CPU, smartphone, smartwatch, tablet, etc., of a server, of a network device, or the like. Based on the instructions, the steps of a method or process for updating the IMD are provided.

The RPE 406 may be in the cloud, in a network, at a server, or the like. The RPE 406 includes a VDE 414 that can simulate the operation of the IMD 408. By simulating the operation of the IMD 408, the VDE 414 obtains information related to the IMD 408, including all updates, changes, modifications, etc. that occur at the IMD 408 over time. The VDE 414 can then store this IMD 408 operational information at a device data store 416. In particular, the RPE 406 includes a data extractor 418 that extracts such information from a transmission data dispatcher 420 that continuously receives transmission data from the IMD 408 via the patient application 412.

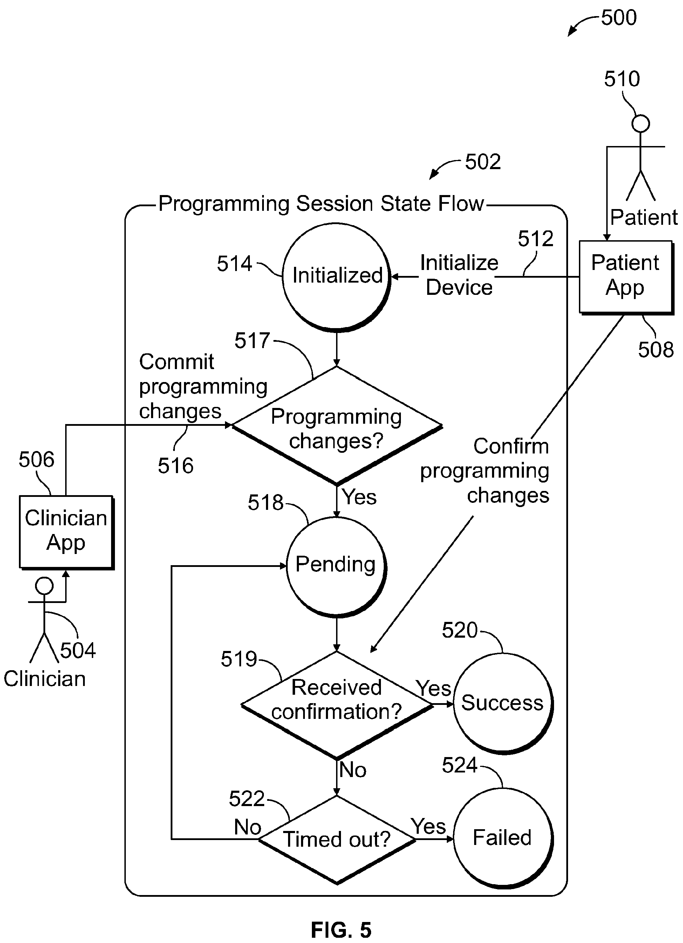

By continuously obtaining the IMD data, the VDE 414 can continuously simulate the operation and device activities of the IMD 408, and store data at the device data store 416. The VDE can thus manage a programming session implemented by the clinician 402 from initialization, to pending, to completed (whether successfully, or failure - see Figure. 5). In this manner, when the clinician app communicates the desired modifications, changes, updates, etc. to the RPE 406, the RPE can synchronize the current state of the IMD 408 with the modification, change, update, etc. desired to be implemented. The VDE 414 can then provide the synchronized data and information to an asynchronous profile generator 422 to generate an asynchronous remote programming profile that includes updated program instructions for updating the IMD 408. In this manner, if the IMD 408 has been modified previously without the knowledge of the clinician 402, the VDE 414 ensures the desired update occurs on the most recent version of a program, software, firmware, application, or the like.

Figure 5 illustrates a block flow diagram 500 of a VDE managing a programming session 502 as implemented by a clinician 504 via a clinician application 506, that in one example is at a clinician programming device. Similar to the embodiment of Figure 4, the clinician programming device may be at a laptop computer, desktop computer, CPU, smart device, tablet, or the like. The clinician application may include instructions executed by one or more processors of the laptop computer, desktop computer, CPU, smart device, Tablet, or the like. Based on the programming session, a patient application 508 of a patient 510 receives updated program instructions for modifying, changing, updating, etc. a treatment, software, firmware, application, program, or the like. The patient application 508 can be at a patient programming device.

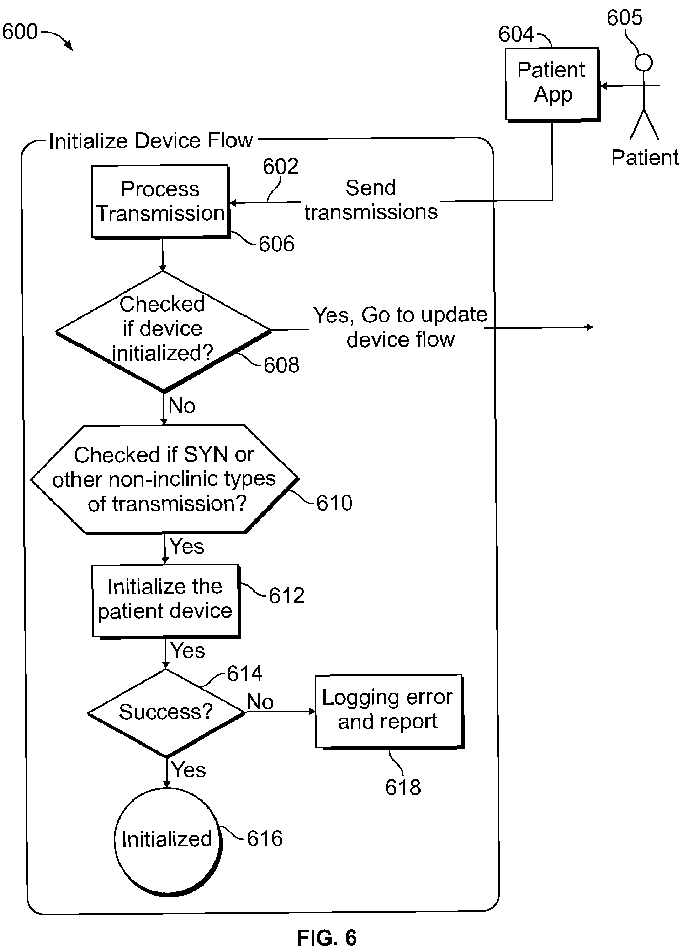

During the programming session 502, at 512, the VDE receives and input from a patient application initializing the IMD device. Thus, at 514 the VDE is initialized and ready for program changes. (See Figure 6). At 516 the VDE receives the requested programming change from the clinician application 506. Then, at 517 a determination is made whether other programming changes have previously been made to the IMD, and in particular whether initialization has occurred. If the patient has not initialized the IMD, the programming changes are not made. In this manner, the VDE prevents the updating of any and all operations, treatments, software, firmware, etc. until initialization has occurred. The initialization ensures the VDE has the most up to date data and information related to the IMD to prevent errors during the update.

If at 517, a determination is made that the IMD is initialized and the changes are appropriate, then the programming changes are implemented to the IMD, and a request for confirmation is provided. At this time, the VDE indicates the changes are pending, and at 518 a pending message may be displayed by the VDE on an electronic device of the patient via the patient application, or on an electronic device of the clinician via the clinician application. In this manner, the clinician and/or patient knows that changes to the IMD are underway.

At 519, while the VDE is still pending, a determination is made regarding whether the confirmation has been received. Once the confirmation of the update, or change is confirmed, then at 520, a success message can be communicated. Again, the success message can be communicated on an electronic device of either the clinician or patient.

If at 519, the VDE is not confirmed, then at 522 a determination is made whether a determined amount of time has lapsed. In particular, an amount of time may be calculated for an update to download, upload, result in a change, or the like. In one example this time may be fifteen minutes. As a result, the determined time may be one hour, such that if confirmation has not occurred in an hour, the update can be considered failed. While in one example one hour can be the determined period, in other examples two hours, a day, a week, etc. can be the determined period. Still, once the determined period is reached without confirmation, at 524 a failure message is provided. The failure message can be displayed at either or both of the clinician electronic device and/or the patient electronic device.

Figure 6 illustrates an example of a process 600 for initializing an IMD in the VDE, and consequently the data store when a patient is not within a clinically setting. Clinical settings can include hospitals, clinics, doctor's offices, treatment centers, or the like. Consequently, the initialization can include a sync-up message, or non-clinic message that is communicated to the RPE, including from a patient application. The message is considered a sync-up message because the message ensures the VDE includes the most recent version of software, firmware, treatment, etc. at the IMD, including any provided in a clinic setting not using the clinician application.

At 602, a communication from a patient application 604 is transmitted to the VDE. In one example, the patient 605 may log into their patient application 604 and actuate an initialization button or key. At 606, the transmission is then processed and at 608, and a determination is made whether the IMD has already been initialized. If the IMD had previously been initialized, such as during an in clinic visit, then no additional steps to be taken for initialization of the IMD.

If at 608, initialization has not previously occurred, then at 610, the VDE checks for sync messages, or other transmissions to the IMD. In particular, the VDE determines if any updates to treatments, software, firmware, or the like has occurred at the IMD without use of the VDE. In this manner, if any such updates have occurred, the VDE can find such transmissions and modify the currently requested update accordingly.

If at 610 a determination is made that no additional transmissions exist, then at 612 initialization of the IMD moves forward with no additional modifications or changes. However, if at 610 additional transmission do exist, then at 612, the initialization of the IMD is based on the requested updates and the additional transmissions. In this manner, the requested updates are made based on the most recent data and information of the IMD.

Once initialization begins at 614, a determination is made whether such initialization was successful. If the initialization is not successful, then at 616 an error is logged by the VDE and an error message is communicated to the clinician application and the patient application. In this manner the patient and clinician understand an initialization failure has occurred and a log is provided for facilitating diagnosis of what caused the error.

If at 614, the initialization is determined to be successful, then at 616 the IMD is initialized. As a result of being initialized, the IMD can now be updated by the clinician application. If at 614 the initialization is determined to not be successful, then at 618 an error is logged and can be communicated to the patient application 604.

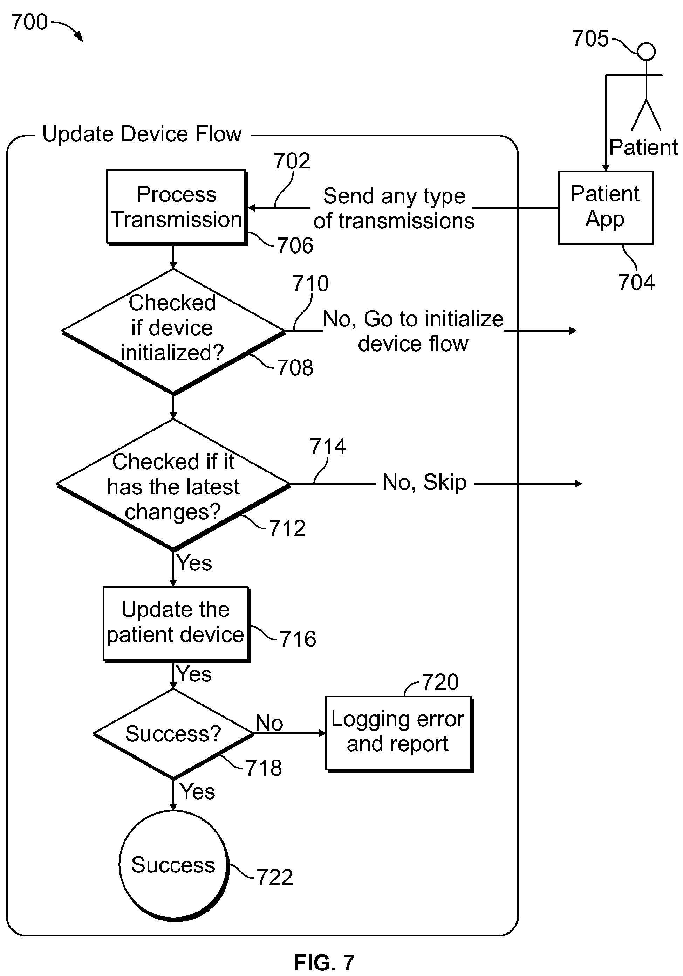

Figure 7 illustrates a block flow diagram of a process 700 of updating an IMD in a clinician setting. Again, the clinician setting may include a hospital, clinic, treatment center, doctor's office, or the like. When the IMD is updated in the clinical setting, such updates need to be communicated to the clinician application such that when an update is desired to be made using the clinician application, the most up to date data and information is available to the clinician application. In addition, any update provided utilizing the clinician application need to be known to the patient application, and IMD, so that changes in the clinic can be made with knowledge of all previous updates.

At 702, the patient application 704 communicates all communication transmissions received at the patient application 704 from a patient 705 to the VDE. At 706, each of the transmissions is processed. During processing, at 708, a determination is made whether the IMD has been initialized. If the device has not yet been initialized, then at 710 steps are taken to initialize the IMD consistent with the steps provided in Figure 5. If at 708 the IMD is initialized, then at 712 a determination is whether the VDE includes the most recent updates, modifications, or changes to the IMD. If the VDE does not have the most recent updates, modification, or changes, then at 714, the IMD is not updated, and instead procedure is provided for receiving the most recent data and information.

If at 714 the most recent updates, modification, or changes are present, then at 716 the VDE updates the IMD. Once the IMD is set to be updated, at 718 a determination is made whether the update is successful. If at 718 the updating is not successful, then at 720 the error is logged, and an error message is communicated. If at 718 the updating is successful, then at 722 a success message is communicated.

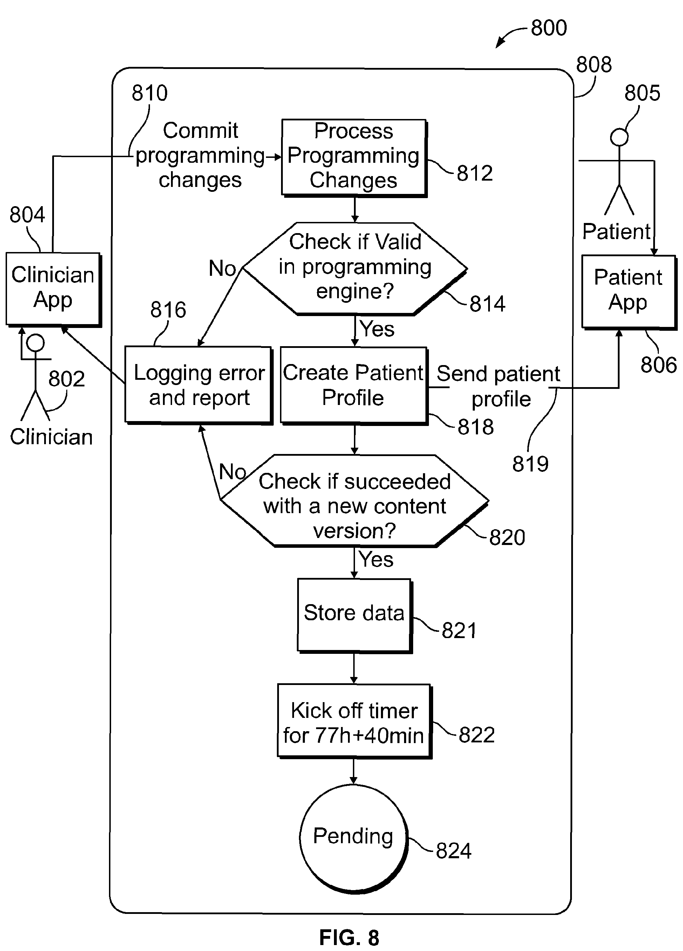

Figure 8 illustrates a block flow diagram for a process 800 where a clinician 802 provides programming updates for an IMD of a patient by providing a communication from a clinician application 804 that is received from a patient 805 via a patient application 806 through a VDE 808.

At the VDE 808, the VDE receives the programming changes 810, and at 812 processes the programing changes virtually. Because the VDE 808 communicates continuously with the patient application 806, when a change, update, etc. occurs at the patient application 806, such a change is communicated to the VDE 808 so that the VDE updates a virtual IMD that is representative of the patient IMD. As a result, the VDE is able to provide verification regarding whether the instructed programming change is allowable, or will accomplish the desired outcome as desired by the clinician 802. In this manner, the VDE provides a check in case the IMD has been updated previously, and/or does not include the same configuration as the clinician application 804 indicates.

Therefore, at 814 a determination is made whether a prospective programing change is valid in the programming engine. If the programming change is not valid, then at 816 an error is generated, logged, and communicated to the clinician application 804. By logging the error and communicating the error to the clinician, the clinician can be made aware a change has previous occurred at the IMD that the clinician was not aware of at the time of the updated program instructions. This allows the clinician to determine the present condition of the IMD, and redetermine whether a change is desired. In one example, after determining the correct status of the IMD, a clinician may decide not to make the update or change previously desire. Alternatively, the clinician can update their programming records, and resubmit the request, only based on the correct version of the software, firmware, treatment schedule, or the like.

If at 814 a determination is made the prospective programming change is valid, then at 818 a patient profile is updated and communicated to the patient application as an asynchronous remote programming profile. If the updated program instructions are the first instructions provided for the IMD, the patient profile can be created. Alternatively, the patient profile is updated with the most recent update to form the asynchronous remote programming profile, and communicated to the patient application at 819 so that the IMD can be updated.

After the patient profile is updated or created at 820 a determination is made whether the new content version was successful. If not, an error is logged and communicated. If at 820 the new content version is successful, the data is stored of the update at 821. In addition, at 822, when the updated program instructions are sent, the VDE automatically begins a timer and at 824 changes status to pending, and a pending message can be provided. In this manner, the VDE continuously checks to verify that the update has occurred. Thus, if another update is sent, and no verification that the update has occurred exists, the VDE can provide such information. Similarly, once the update is completed the message may be communicated to update the status of the VDE to provide a similar updated status. Alternatively, if the timer expires without receiving an acknowledgement message, a failure message can be displayed at a clinician programming device or patient programming device to alert the clinician, and/or patient that the update has not occurred.

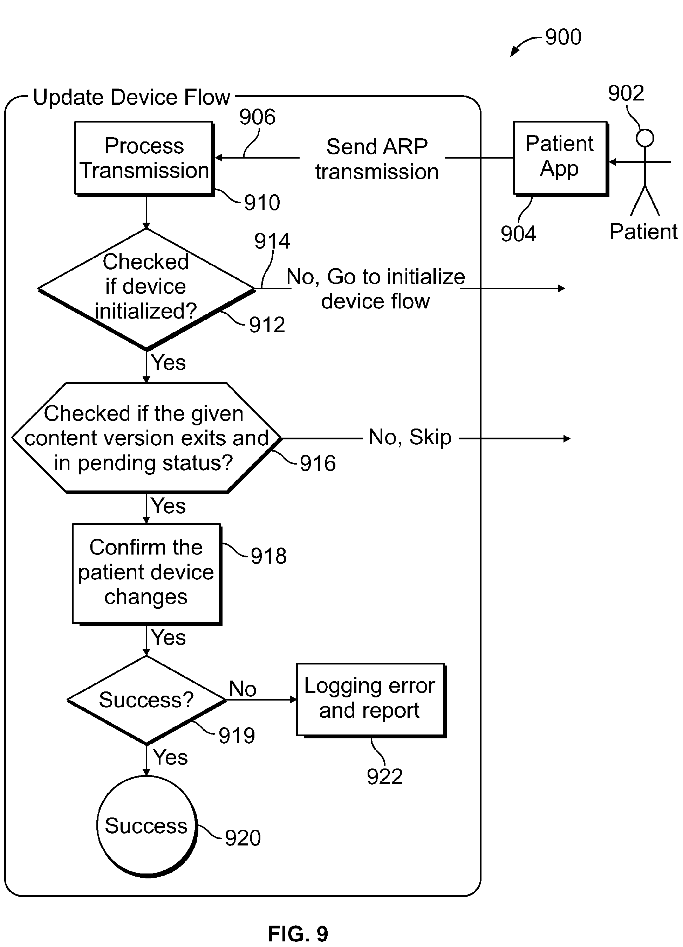

Figure 9 illustrates a process 900 for updating an IMD of a patient 902 using a patient application 904. In example embodiments, any of the previous systems, devices, methods, processes, etc. may be utilized to provide the update.

At 906 the patient application 904 transmits a message that includes an update to the IMD. At 910, the message is processed the VDE. After the message is process and a determination is made that the patient desires to make changes, update, etc. the IMD, at 912 a determination is made whether the IMD has be initialized for updating the IMD. Again, similar to other processes, the VDE also insures the IMD is initialized before any additional changes, updates, etc. can occur. If at 912 initialization has not previous occurred, then a message 914 is communicated to start the initialization process. If initialization has occurred, then at 916 a determination is made to see if an update is currently pending or if the content of the update already exists. If the content does not exist, or is not pending, then the rest of the process 900 can be skipped.

If at 916 the content exists, or is pending, then at 918 confirmation is requested by the VDE. In particular, the VDE verifies that the desired update, change, etc. has occurred. Therefore, at 919, a determination is made whether the update was successful. When successful, an acknowledgement message is communicated to the VDE at 920. In another example, a success message is displayed in response to receiving the acknowledgment message. If at 919 the update is not successful, then at 922 an error or failure message is communicated, and the error is logged.

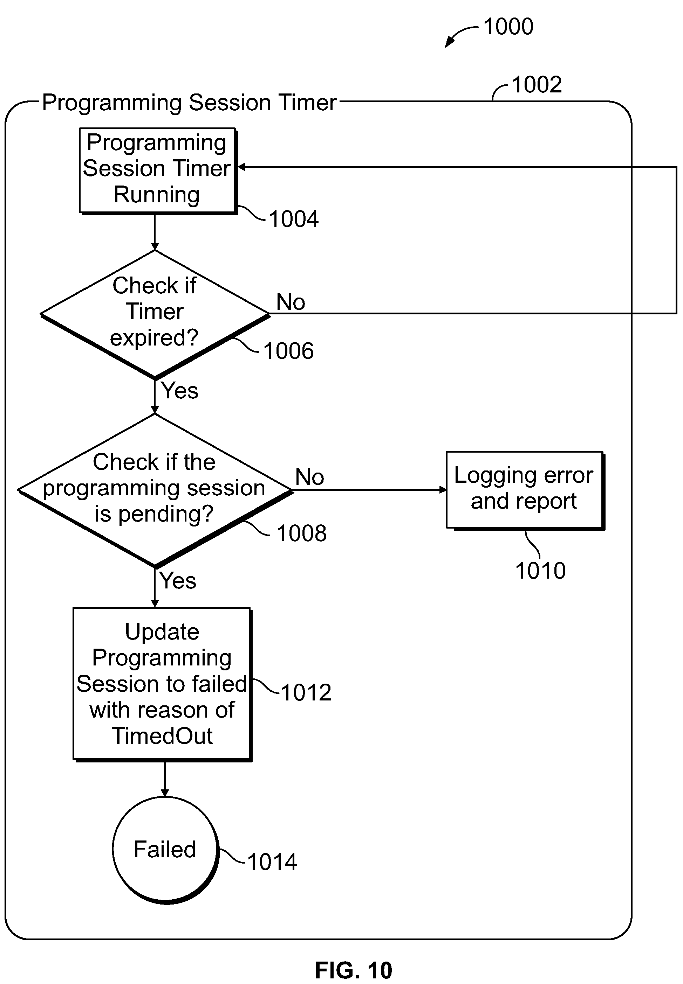

Figure 10 illustrates a flow block diagram of a process 1000 that utilizes a program timer 1002 of a VDE to determine if an update, change, or the like has been implemented at an IMD. At 1004 the program timer begins running during a session. In one example, a clinician application is utilized to request a software update for the IMD. Upon verifying initialization of the IMD, the session timer begins to run for a determined period. The determined period can be determined based on the amount expected time for a change, update, etc. to occur. So, in an instance where software is being updated and is expected to take approximately one hour, the determined period can be two hours. In this manner, the determined period can be based on the expected time to complete a task, change, update, or the like. Alternatively, the determined period can be a static period of time, such as one day. Thus, regardless of the size of the update, whether expected to take five minutes, or five hours, the determined period can be a day. In other examples, the determined period can be two days, twelve hours, two hours, one week, etc. Still, the determined period begins when the change, update, etc. begins.

At 1006, a determination is made whether the timer has expired. The program session timer in one example is configured to check to see if the determined period has expired based on pre-set intervals. In one example, the pre-set interval can be one hour. Alternatively the pre-set interval can be five minutes, two hours, etc. In particular, if the timer has expired, an error message may automatically be transmitted to notify a clinician, patient, etc. that the update was unsuccessful.

If the timer has expired, then at 1008 a determination is made whether a program session is still pending (e.g. whether an acknowledgement message has been received). So, if no acknowledgement message of completion of an update has been received by the VDE, the program session remains pending, the VDE continues to wait to receive the verification. Once the determined period has lapsed, if no acknowledgement message has been received, and the program session is no longer pending, at 1010 an error or failure message is logged into the data store, and communicated to the clinician and/or patient. Without a confirmation, an error has occurred, and the failure message is communicated to provide notice to the clinician or patient. Alternatively, if the VDE is still pending at 1008, then at 1012 an updating failed message 1014 is communicated to either the patient and/or the clinician. Basically, after the determined period has lapsed with the status still pending, an assessment can be made that the update has failed.

### Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the figures, which illustrate example methods, devices, and program products according to various example embodiments. The program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims.

## Claims

1. A medical device assembly comprising:
a remote programming engine (406);
a clinician programming device (14, 104) configured to communicate with an implanted medical device (12, 102, 408) of a patient (8);
the clinician programming device (14, 104) including one or more processors (116) configured to communicate program instructions to the remote programming engine (406),
**characterized in that** the remote programming engine (406) comprises one or more processors (414, 422) configured to:
simulate operation of the implanted medical device (12, 102, 408) based on the program instructions; and
communicate updated program instructions to the implanted medical device (12, 102, 408) based on the operation simulated.

2. The medical device assembly of claim 1, wherein to simulate operation of the implanted medical device (12, 102, 408), the one or more processors (414) of the remote programming engine (406) are further configured to determine initialization of the programming session, whether the programming session is pending, and termination of the programming session.

3. The medical device assembly of claim 2, wherein the one or more processors (414) of the remote programming engine (406) are further configured to determine whether the termination of the programming session is in response to completion of the programming session, or in response to a programming error.

4. The medical device assembly of claim 2 or 3, wherein to determine initialization of the programming session, the one or more processors (414, 422) of the remote programming engine (406) are further configured to:
generate an asynchronous remote programming profile that includes the updated program instructions; and
communicate the asynchronous remote programing profile to the implanted medical device (12, 102, 408).

5. A computer program product comprising a non-signal computer readable storage medium comprising computer executable code to:
obtain program instructions related to a programming session of an implanted medical device (12, 102, 408), **characterized in that** the non-signal computer readable storage medium comprises computer executable code to:
simulate operation of the implanted medical device (12, 102, 408) based on the program instructions; and
communicate updated program instructions to the implanted medical device (12, 102, 408) based on the operation simulated.

6. The computer program product of claim 5, wherein to simulate operation of the implanted medical device (12, 102, 408), the computer executable code is configured to determine initialization of the programming session, whether the programming session is pending, and termination of the programming session.

7. The computer program product of claim 6 further comprising computer executable code to determine whether the termination of the programming session is in response to completion of the programming session, or in response to a programming error.

8. The computer program product of any one of claims 5 to 7, wherein to determine initialization of the programming session, the computer executable code is configured to:
access a patient profile at a virtual device engine, VDE, (414) in response to obtaining the program instructions;
generate an asynchronous remote programming profile that includes the updated program instructions; and
communicate the asynchronous remote programing profile to the implanted medical device (12, 102, 408).

9. The computer program product of claim 8, the computer executable code is configured to:
generate a pending message in response to communicating the asynchronous remote programing profile; and
present the pending message until an acknowledgment message is received from a patient application (412).

10. The computer program product of claim 9, the computer executable code is configured to communicate a success message to a clinician application in response to receiving the acknowledgment message.

11. A clinician programming device (14, 104) configured to communicate with an implanted medical device (12, 102, 408) of a patient (8), wherein the clinician programming device (14, 104) includes one or more processors (116) configured to:
communicate program instructions related to a programming session of the implanted medical device (12, 102, 408) to a remote programming engine (406), **characterized in that** the one or more processors (116) are configured to:
receive updated program instructions from the remote programming engine (406) based on a simulation of operation of the implanted medical device (12, 102, 408) based on the program instructions; and
communicate the updated program instructions to the implanted medical device (12, 102, 408).

12. The medical device assembly of claim 11, wherein the one or more processors (116) are configured to receive communication related to initialization of the implantable medical device (12, 102, 408), related to whether the programming session is pending, and related to termination of the programming session.

13. The medical device assembly of claim 12, wherein the one or more processors (116) are further configured to determine whether the termination of the programming session is in response to completion of the programming session, or in response to a programming error.

14. The medical device assembly of claim 12 or 13, wherein the one or more processors (116) are further configured to:
receive a message from a virtual device engine, VDE, (414), the message including program instructions for the programming session that is related to a treatment to be performed by the implanted medical device (12, 102, 408);
receive an asynchronous remote programming profile from the VDE (414) that includes the updated program instructions; and
communicate the asynchronous remote programming profile to the implanted medical device (12, 102, 408).

15. The medical device assembly of claim 14, the one or more processors (116) being further configured to:
generate and display a pending message in response to communicating the asynchronous remote programming profile; and
present the pending message until an acknowledgment message is received from a patient application (412).

## Patentansprüche

1. Baugruppe für medizinische Vorrichtungen, umfassend:
eine Fernprogrammier-Engine (406);
eine Kliniker-Programmiervorrichtung (14, 104), die dazu konfiguriert ist, mit einer implantierten medizinischen Vorrichtung (12, 102, 408) eines Patienten (8) zu kommunizieren;
wobei die Kliniker-Programmiervorrichtung (14, 104) einen oder mehrere Prozessoren (116) beinhaltet, der/die dazu konfiguriert ist/sind, Programmanweisungen an die Fernprogrammier-Engine (406) zu kommunizieren,
**dadurch gekennzeichnet, dass** die Fernprogrammier-Engine (406) einen oder mehrere Prozessoren (414, 422) umfasst, der/die zu Folgendem konfiguriert ist/sind:
Simulieren des Betriebs der implantierten medizinischen Vorrichtung (12, 102, 408) basierend auf den Programmanweisungen; und
Kommunizieren der aktualisierten Programmanweisungen an die implantierte medizinische Vorrichtung (12, 102, 408) basierend auf dem simulierten Betrieb.

2. Baugruppe für medizinische Vorrichtungen nach Anspruch 1, wobei, um den Betrieb der implantierten medizinischen Vorrichtung (12, 102, 408) zu simulieren, der eine oder die mehreren Prozessoren (414) der Fernprogrammier-Engine (406) ferner dazu konfiguriert ist/sind, eine Initialisierung der Programmiersitzung, ob die Programmiersitzung ausstehend ist und eine Beendigung der Programmiersitzung zu bestimmen.

3. Baugruppe für medizinische Vorrichtungen nach Anspruch 2, wobei der eine oder die mehreren Prozessoren (414) der Fernprogrammier-Engine (406) ferner dazu konfiguriert ist/sind, zu bestimmen, ob die Beendigung der Programmiersitzung als Reaktion auf einen Abschluss der Programmiersitzung oder als Reaktion auf einen Programmierfehler erfolgt.

4. Baugruppe für medizinische Vorrichtungen nach Anspruch 2 oder 3, wobei, um die Initialisierung der Programmiersitzung zu bestimmen, der eine oder die mehreren Prozessoren (414, 422) der Fernprogrammier-Engine (406) ferner zu Folgendem konfiguriert ist/sind:
Generieren eines asynchronen Fernprogrammierprofils, das die aktualisierten Programmanweisungen beinhaltet; und
Kommunizieren des asynchronen Fernprogrammierprofils an die implantierte medizinische Vorrichtung (12, 102, 408).

5. Computerprogrammprodukt, umfassend ein signalfreies computerlesbares Speichermedium, das computerausführbaren Code für Folgendes umfasst:
Erlangen von Programmanweisungen in Bezug auf eine Programmiersitzung einer implantierten medizinischen Vorrichtung (12, 102, 408), **dadurch gekennzeichnet, dass** das signalfreie computerlesbare Speichermedium computerausführbaren Code für Folgendes umfasst:
Simulieren des Betriebs der implantierten medizinischen Vorrichtung (12, 102, 408) basierend auf den Programmanweisungen; und
Kommunizieren der aktualisierten Programmanweisungen an die implantierte medizinische Vorrichtung (12, 102, 408) basierend auf dem simulierten Betrieb.

6. Computerprogrammprodukt nach Anspruch 5, wobei, um den Betrieb der implantierten medizinischen Vorrichtung (12, 102, 408) zu simulieren, der computerausführbare Code dazu konfiguriert ist, eine Initialisierung der Programmiersitzung, ob die Programmiersitzung ausstehend ist und eine Beendigung der Programmiersitzung zu bestimmen.

7. Computerprogrammprodukt nach Anspruch 6, ferner umfassend computerausführbaren Code, um zu bestimmen, ob die Beendigung der Programmiersitzung als Reaktion auf einen Abschluss der Programmiersitzung oder als Reaktion auf einen Programmierfehler erfolgt.

8. Computerprogrammprodukt nach einem der Ansprüche 5 bis 7, wobei, um die Initialisierung der Programmiersitzung zu bestimmen, der computerausführbare Code zu Folgendem konfiguriert ist:
Zugreifen auf ein Patientenprofil bei einer Engine für virtuelle Vorrichtungen, VDE, (414), als Reaktion auf das Erlangen der Programmanweisungen;
Generieren eines asynchronen Fernprogrammierprofils, das die aktualisierten Programmanweisungen beinhaltet; und
Kommunizieren des asynchronen Fernprogrammierprofils an die implantierte medizinische Vorrichtung (12, 102, 408).

9. Computerprogrammprodukt nach Anspruch 8, wobei der computerausführbare Code zu Folgendem konfiguriert ist:
Generieren einer ausstehenden Nachricht als Reaktion auf das Kommunizieren des asynchronen Fernprogrammierprofils; und
Darstellen der ausstehenden Nachricht, bis eine Bestätigungsnachricht von einer Patienten-Anwendung (412) empfangen wird.

10. Computerprogrammprodukt nach Anspruch 9, wobei der computerausführbare Code dazu konfiguriert ist, als Reaktion auf Empfangen der Bestätigungsnachricht eine Erfolgsnachricht an eine Kliniker-Anwendung zu kommunizieren.

11. Kliniker-Programmiervorrichtung (14, 104), die dazu konfiguriert ist, mit einer implantierten medizinischen Vorrichtung (12, 102, 408) eines Patienten (8) zu kommunizieren, wobei die Kliniker-Programmiervorrichtung (14, 104) einen oder mehrere Prozessoren (116) beinhaltet, der/die zu Folgendem konfiguriert ist/sind:
Kommunizieren von Programmanweisungen in Bezug auf eine Programmiersitzung der implantierten medizinischen Vorrichtung (12, 102, 408) an eine Fernprogrammier-Engine (406), **dadurch gekennzeichnet, dass** der eine oder die mehreren Prozessoren (116) zu Folgendem konfiguriert ist/sind:
Empfangen von aktualisierten Programmanweisungen von der Fernprogrammier-Engine (406) basierend auf einer Simulation des Betriebs der implantierten medizinischen Vorrichtung (12, 102, 408) basierend auf den Programmanweisungen; und
Kommunizieren der aktualisierten Programmanweisungen an die implantierte medizinische Vorrichtung (12, 102, 408).

12. Baugruppe für medizinische Vorrichtungen nach Anspruch 11, wobei der eine oder die mehreren Prozessoren (116) dazu konfiguriert ist/sind, Kommunikation in Bezug auf eine Initialisierung der implantierbaren medizinischen Vorrichtung (12, 102, 408), in Bezug darauf, ob die Programmiersitzung ausstehend ist, und in Bezug auf eine Beendigung der Programmiersitzung zu empfangen.

13. Baugruppe für medizinische Vorrichtungen nach Anspruch 12, wobei der eine oder die mehreren Prozessoren (116) ferner dazu konfiguriert ist/sind, zu bestimmen, ob die Beendigung der Programmiersitzung als Reaktion auf einen Abschluss der Programmiersitzung oder als Reaktion auf einen Programmierfehler erfolgt.

14. Baugruppe für medizinische Vorrichtungen nach Anspruch 12 oder 13, wobei der eine oder die mehreren Prozessoren (116) ferner zu Folgendem konfiguriert ist/sind:
Empfangen einer Nachricht von einer Engine für virtuelle Vorrichtungen, VDE, (414), wobei die Nachricht Programmanweisungen für die Programmiersitzung, die sich auf eine durch die implantierte medizinische Vorrichtung (12, 102, 408) durchzuführende Behandlung bezieht, beinhaltet;
Empfangen eines asynchronen Fernprogrammierprofils von der VDE (414), das die aktualisierten Programmanweisungen beinhaltet; und
Kommunizieren des asynchronen Fernprogrammierprofils an die implantierte medizinische Vorrichtung (12, 102, 408).

15. Baugruppe für medizinische Vorrichtungen nach Anspruch 14, wobei der eine oder die mehreren Prozessoren (116) ferner zu Folgendem konfiguriert ist/sind:
Generieren und Anzeigen einer ausstehenden Nachricht als Reaktion auf das Kommunizieren des asynchronen Fernprogrammierprofils; und
Darstellen der ausstehenden Nachricht, bis eine Bestätigungsnachricht von einer Patienten-Anwendung (412) empfangen wird.

## Revendications

1. Ensemble de dispositif médical comprenant :
un moteur de programmation à distance (406) ;
un dispositif de programmation de clinicien (14, 104) configuré pour communiquer avec un dispositif médical implanté (12, 102, 408) d'un patient (8) ;
le dispositif de programmation de clinicien (14, 104) comprenant un ou plusieurs processeurs (116) configurés pour communiquer des instructions de programme au moteur de programmation à distance (406), **caractérisé en ce que** le moteur de programmation à distance (406) comprend un ou plusieurs processeurs (414, 422) configurés pour :
simuler le fonctionnement du dispositif médical implanté (12, 102, 408) sur la base des instructions de programme ; et
communiquer des instructions de programme mises à jour au dispositif médical implanté (12, 102, 408) en fonction de l'opération simulée.

2. Ensemble de dispositif médical selon la revendication 1, dans lequel pour simuler le fonctionnement du dispositif médical implanté (12, 102, 408), les un ou plusieurs processeurs (414) du moteur de programmation à distance (406) sont en outre configurés pour déterminer l'initialisation de la session de programmation, si la session de programmation est en attente, et la fin de la session de programmation.

3. Ensemble de dispositif médical selon la revendication 2, dans lequel les un ou plusieurs processeurs (414) du moteur de programmation à distance (406) sont en outre configurés pour déterminer si la fin de la session de programmation est en réponse à l'achèvement de la session de programmation, ou en réponse à une erreur de programmation.

4. Ensemble de dispositif médical selon la revendication 2 ou 3, dans lequel, pour déterminer l'initialisation de la session de programmation, les un ou plusieurs processeurs (414, 422) du moteur de programmation à distance (406) sont en outre configurés pour :
générer un profil de programmation à distance asynchrone qui inclut les instructions de programme mises à jour ; et
communiquer le profil de programmation à distance asynchrone au dispositif médical implanté (12, 102, 408).

5. Produit de programme informatique comprenant un support de stockage lisible par ordinateur sans signal comprenant un code exécutable par ordinateur pour :
obtenir des instructions de programme relatives à une session de programmation d'un dispositif médical implanté (12, 102, 408),
**caractérisé en ce que** le support de stockage lisible par ordinateur sans signal comprend un code exécutable par ordinateur pour :
simuler le fonctionnement du dispositif médical implanté (12, 102, 408) sur la base des instructions de programme ; et
communiquer des instructions de programme mises à jour au dispositif médical implanté (12, 102, 408) en fonction de l'opération simulée.

6. Produit de programme informatique selon la revendication 5, dans lequel pour simuler le fonctionnement du dispositif médical implanté (12, 102, 408), le code exécutable par ordinateur est configuré pour déterminer l'initialisation de la session de programmation, si la session de programmation est en attente, et la fin de la session de programmation.

7. Produit de programme informatique selon la revendication 6 comprenant en outre un code exécutable par ordinateur pour déterminer si la fin de la session de programmation est en réponse à l'achèvement de la session de programmation ou en réponse à une erreur de programmation.

8. Produit de programme informatique selon l'une quelconque des revendications 5 à 7, dans lequel, pour déterminer l'initialisation de la session de programmation, le code exécutable de l'ordinateur est configuré pour :
accéder à un profil de patient au niveau d'un moteur de dispositif virtuel, VDE, (414) en réponse à l'obtention des instructions de programme :
générer un profil de programmation à distance asynchrone qui inclut les instructions de programme mises à jour ; et
communiquer le profil de programmation à distance asynchrone au dispositif médical implanté (12, 102, 408).

9. Produit de programme informatique selon la revendication 8, le code exécutable par ordinateur est configuré pour :
générer un message en attente en réponse à la communication du profil de programmation à distance asynchrone ; et
présenter le message en attente jusqu'à ce qu'un message d'accusé de réception soit reçu à partir d'une application patient (412).

10. Produit de programme informatique selon la revendication 9, le code exécutable par ordinateur est configuré pour communiquer un message de réussite à une application de clinicien en réponse à la réception du message d'accusé de réception.

11. Dispositif de programmation de clinicien (14, 104) configuré pour communiquer avec un dispositif médical implanté (12, 102, 408) d'un patient (8), le dispositif de programmation de clinicien (14, 104) comprenant un ou plusieurs processeurs (116) configurés pour :
communiquer des instructions de programme relatives à une session de programmation du dispositif médical implanté (12, 102, 408) à un moteur de programmation à distance (406), **caractérisé en ce que** les un ou plusieurs processeurs (116) sont configurés pour :
recevoir des instructions de programme mises à jour du moteur de programmation à distance (406) sur la base d'une simulation du fonctionnement du dispositif médical implanté (12, 102, 408) sur la base des instructions de programme ; et
communiquer les instructions de programme mises à jour au dispositif médical implanté (12, 102, 408).

12. Ensemble de dispositif médical selon la revendication 11, dans lequel les un ou plusieurs processeurs (116) sont configurés pour recevoir une communication relative à l'initialisation du dispositif médical implantable (12, 102, 408), relative au fait que la session de programmation est en attente et relative à la fin de la session de programmation.

13. Ensemble de dispositif médical selon la revendication 12, dans lequel les un ou plusieurs processeurs (116) sont en outre configurés pour déterminer si la fin de la session de programmation est en réponse à l'achèvement de la session de programmation, ou en réponse à une erreur de programmation.

14. Ensemble de dispositif médical selon la revendication 12 ou 13, dans lequel les un ou plusieurs processeurs (116) sont en outre configurés pour :
recevoir un message à partir d'un moteur de dispositif virtuel, VDE, (414), le message comprenant des instructions de programme pour la session de programmation qui est liée à un traitement à effectuer par le dispositif médical implanté (12, 102, 408) ;
recevoir un profil de programmation à distance asynchrone à partir du VDE (414) qui comprend les instructions de programme mises à jour ; et
communiquer le profil de programmation à distance asynchrone au dispositif médical implanté (12, 102, 408).

15. Ensemble de dispositif médical selon la revendication 14, les un ou plusieurs processeurs (116) étant en outre configurés pour :
générer et afficher un message en attente en réponse à la communication du profil de programmation à distance asynchrone ; et
présenter le message en attente jusqu'à ce qu'un message d'accusé de réception soit reçu à partir d'une application patient (412).
